(19) Europäisches Patentamt
European Patent Office
Office européen des brevets

(11) Publication number: **0 318 914 B1**

(12) **EUROPEAN PATENT SPECIFICATION**

(45) Date of publication of patent specification: **08.06.94**

(51) Int. Cl.5: **C12N 1/00**, C12P 17/00, C12N 1/38, //(C12P17/02,7:00, 11:00,13:00)

(21) Application number: **88119872.5**

(22) Date of filing: **29.11.88**

(54) **Method for regenerating deactivated microorganisms.**

(30) Priority: **30.11.87 JP 302584/87**
**17.12.87 JP 319832/87**
**17.12.87 JP 319833/87**
**17.12.87 JP 319834/87**
**27.05.88 JP 128242/88**

(43) Date of publication of application:
**07.06.89 Bulletin 89/23**

(45) Publication of the grant of the patent:
**08.06.94 Bulletin 94/23**

(84) Designated Contracting States:
**BE CH DE FR GB IT LI NL SE**

(56) References cited:
**EP-A- 042 306**
**EP-A- 098 138**
**EP-A- 178 153**
**FR-A- 2 125 563**
**GB-A- 2 018 822**

**PATENT ABSTRACTS OF JAPAN, vol. 2, no. 52, (C-78) (184), 14 April 1978**

(73) Proprietor: **IDEMITSU KOSAN COMPANY LIMITED**
**1-1, Marunouchi 3-chome**
**Chiyoda-ku**
**Tokyo 100(JP)**

(72) Inventor: **Suzuki, Motoshi**
**14 Bertie Road**
**Kenilworth Warwickshire(GB)**
Inventor: **Dalton, Howard**
**32 Southam Road**
**Radford Semele**
**Leamington Spa Warwickshire(GB)**
Inventor: **Richards, Anthony O'Leary**
**27 Warwick place**
**Leamington Spa Warwickshire(GB)**
Inventor: **Stanley, Stephen Hall**
**12 The Gardens**
**Kenilworth Warwickshire(GB)**

(74) Representative: **VOSSIUS & PARTNER**
**Postfach 86 07 67**
**D-81634 München (DE)**

**EP 0 318 914 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for the reactivation of deactivated microbial cells and, more particularly, to a method for the reactivation of an activity of a methane monooxygenase of methane-utilizing bacteria which have partially or totally lost their capability of oxidizing methane, by reactivating them in a reactivation solution to which at least one specific substance is added.

The methane oxidation enzymes (methane monooxygenase) of methane-utilizing bacteria cannot only oxidize methane but also alkanes, alkenes, cyclic compounds, organic sulfur compounds and organic nitrogen compounds to the corresponding oxides. Therefore, these bacteria are of high industrial value, but have the disadvantage that they tend to be easily deactivated due to their low stability. If the capability of oxidizing methane of deactivated microorganisms can be reactivated, it is possible to repeatedly use the microbial cells and, hence, cut down the production costs. Until now, it has been known to regenerate the capability of oxidizing methane of deactivated microbial cells by the supply of a carbon source and oxygen. However, so far no satisfactory results were obtained (U.S. Pat. No. 4,348,476).

Therefore, the technical problem underlying the present invention is to provide a method for reactivating an activity of a methane monooxygenase of methane-utilizing bacteria which have partially or totally lost their capability of oxidizing methane to a sufficient level, which permits to use them repeatedly. The solution of this technical problem is achieved by providing a method capable of reactivating an activity of a methane monooxygenase of methane-utilizing bacteria which have partially or totally lost their capability of oxidizing methane wherein they are regenerated in a reactivation solution to which at least one specific substance is added.

The present invention relates to a method for reactivating an activity of a methane monooxygenase of methane-utilizing bacteria which have partially or totally lost their capability of oxidizing methane wherein said methane-utilizing bacteria are cultured in a reactivation solution containing at least one of the compounds methane, methanol and formaldehyde, while supplying a nitrogen source, a sulfur source and oxygen to the reactivation solution.

In a preferred embodiment of the method of the present invention oxygen and a methionine derivative are supplied to the reactivation solution.

Furthermore, the present invention relates to a method for the continuous production of oxides which comprises contacting methane-utilizing bacteria having a methane monooxygenase in a reactor vessel with alkanes, alkenes or cyclic compounds in the presence of an electron donor, discharging the resulting oxides from the system to the outside, regenerating said methane-utilizing bacteria having a decreased capability of oxidizing methane according to the above methods in order to recover their capability of oxidizing methane and then returning said bacteria into the reactor vessel for the further production of oxides.

Figures 1 and 2 each are a flow sheet illustrating the embodiments of the apparatus systems used for carrying out the method of the present invention.

A: Reactor Vessel

B: Regenerating Vessel

C, D: Scrubbers

An oxidation reaction making use of methane-utilizing bacteria is generally carried out by contacting them with a starting material in the presence of an electron donor.

Methane-utilizing bacteria which are useful in the method of the present invention preferably belong to the genera Methylococcus, such as Methylococcus capsulatus NCIB 11132, Methylomonas, such as Methylomonas agile NCIB 11124, Methylosinus, such as Methylosinus trichlosporium NCIB 11131, Methylocystis, such as Methylocystis parvus NCIB 11129, Methylobacter, such as Methylobacter capsulata NCIB 11128 or Methylobacterium, such as Methylobacterium organophilum ATCC 27886.

The media used for culturing the aforesaid methane-utilizing bacteria may be such that they permit sufficient propagation of said bacteria, and methane, methanol and the like may usually be used as the carbon source. The nitrogen source used may be ammonium chloride, pottasium nitrate, ammonium nitrate and the like which are ordinarily used in the prior art. Besides, phosphates, calcium salts, magnesium salts and slight amounts of inorganic salts (cupric salts, ferrous salts, cobalt salts, etc.) may optionally be added. Preferably, use may be made of the culture medium of Whittenbury et al. (J. Gen. Microbiol., 61, pp. 205-208, 1970). A culture vessel containing a culture medium is internally replaced by a mixed gas of methane and an oxygenous gas (e.g. air), and the methane-utilizing bacteria are inoculated into the culture medium in contact with said gas.

The methane-utilizing bacteria are aerobic microorganisms, and may be cultured batchwise or continuously at 15 to 60 °C, preferably at 20 ° to 50 °C under aerobic conditions.

The cultured product may be used as such for the oxidation of the raw materials to be described later. However, microbial masses obtained by solid/liquid separation by means such as centrifuging may also be used. Further, the obtained microbial masses may be washed with and suspended in a suitable solution such as a phosphate buffer. For use the microbial masses may also be immobilized in the conventional manner.

It is required that, in a reaction tank, the aforesaid methane-utilizing bacteria are contacted with the raw material in the presence of an electron donor. The electron donor used includes methane; lower alcohols such as methyl alcohol or ethyl alcohol; $\alpha,\omega$-diols such as ethylene glycol or 1,4-butanediol; lower aldehydes such as formaldehyde, acetoaldehyde or propionaldehyde, formates such as formic acid or sodium formate; hydrogen; $NADH_2$; $NADPH_2$ and methane. These may be used alone or in combination.

The raw materials used may include alkanes such as methane, ethane, propane, butane, hexane or octane; alkenes such as ethylene, propylene or butenes; cyclic compounds such as cyclohexane, benzene or toluene or their derivatives (for instance, halogen-, nitro- and amino-substituted derivatives, alcohols, ethers and esters).

The catalytic oxidation reactions of the raw materials with the methane-utilizing bacteria may be carried out in the presence of electron donors, and the reaction temperature and time may be determined in such a manner that the desired oxidation reactions occur satisfactorily, taking the types of the raw materials and methane-utilizing bacteria into consideration. The reaction conditions may generally be pH 5.5 to 9.0, preferably 6.0 to 8.5 and a temperature of 15 to 60 °C, preferably 20 to 50 °C.

The corresponding epoxides, alcohols, aldehydes, S-oxides, N-oxides and the like are produced in these oxidation reactions.

It is required to rapidly remove the resulting oxide from the reactor vessel. For instance, a part of the microorganism-containing reaction mixture is introduced into a product collector such as a scrubber, an extractor or a membrane separator to remove the oxides. The whole or a part of the reaction mixture, from which the oxide has been removed, is returned into the reactor vessel.

The methane-utilizing bacteria used in the oxidation reactions as mentioned above partly or wholly lose their methane oxidizability upon repeated use, resulting in deactivated microbial cells. Such microbial cells cannot be re-used directly for the oxidation reactions. In order to regenerate or reactivate the microorganisms which have lost their capability of oxidizing methane, they are taken out of the reaction vessel and supplied to the regenerating vessel. Thereafter, the regeneration or reactivation operation may be carried out, while supplying thereto a carbon source, a nitrogen source, a sulfur source and oxygen. The carbon sources to be used include methane, methanol and formaldehyde, which may be used alone or in combination. The amount of methanol or formaldehyde added is in the range of 10 to 600 nmol per minute•mg of microbial cells, preferably 30 to 400 nmol/min.•mg of microbial cells. Methanol or formaldehyde may be supplied at once or continuously. However, it is preferred that they are continuously supplied in suitable amounts. This is because when a large amount of methanol or formaldehyde is added at once, the desired reactivation may not take place due to their toxicity. When methanol or formaldehyde is continuously supplied, the microbial cells may be reactivated even in an amount of below 10 nmol/min.•mg of microbial cells, but considerable time is required for that purpose. When methanol or formaldehyde is supplied in an amount of larger than 600 nmol/min.•mg of microbial cells, on the other hand, the reactivation of the microbial cells may be interrupted, since it is not completely consumed and accumulated. A mixed gas of methane and air is used, since the methane-utilizing bacteria are aerobic bacteria. In this case, the amount of methane to be supplied is equal to or larger than 10 nmol/min.•mg of microbial cells, preferably 30 nmol/min.•mg of microbial cells. No particular limitation is imposed upon the volume ratio of methane to air. It is understood, however, that the reactivation of microorganisms is retarded in a state extremely deficient in oxygen, and does not take place at all in the absence of oxygen. Too excessive supply of methane may not hinder the reactivation of microorganisms, but results in waste, since the microorganisms cannot consume methane in an amount larger than required. A methane supply rate and a methane/air mixing ratio sufficient to permit consumption of methane in an amount of 30 nmol/min.•mg of microbial cells may be used. The nitrogen sources to be used may include inorganic and organic ones such as gaseous nitrogen, nitric acid, potassium nitrate, sodium nitrate, ammonium nitrate, ammonium sulfate, peptone, casaminoic acid, L-glutamine and L-asparagine, which may be added in an amount of not less than 1 nmol/min.•mg of microbial cells, preferably in a range of 2 to 500 nmol/min.•mg of microbial cells. The reactivation of the microorganism is retarded, when the amount of the nitrogen source is smaller. Excessive addition of the nitrogen source has not any appreciable effect, but may rather inhibit the reactivation of the microorganism.

The sulfur sources to be used may include sulfuric acid, magnesium sulfate, potassium sulfate, sodium sulfate, sodium sulfide, hydrosulfide and sodium sulfhydrate, which may be added in an amount of 0.02

nmol/min.•mg of microbial cells or larger, preferably in a range of 0.1 to 150 nmol/min.•mg of microbial cells. The activation of microbial cells is retarded, when the amount of the sulfur source is smaller, whereas excessive addition of the sulfur source has not any appreciable effect. These components may continuously be supplied in a certain proportion per a certain time, or may alternatively be added at once in an amount corresponding to several hours at the initiation time of reactivation or in the course of reactivation. When the components are added at once, they may be supplied in an amount corresponding to the amount thereof supplied in the aforesaid continuous manner. When the regeneration of microorganisms is carried out, the sufficient addition of phosphoric acid, magnesium and trace metals in addition to the above described components may achieve the regeneration of microorganisms simultaneously with the propagation of microorganisms. It is to be noted that an insufficient amount of oxygen results in a delay in the regeneration of microorganisms. The reactivation of microorganism may be carried out by shaking the culture at 20 to 50 °C under aerobic conditions, since the methane-utilizing bacteria are aerobic. Although the reactivation temperatures vary with microorganisms, sufficient activation of microbial cells may be achievable in a temperature range in which the microbial cells can at least grow. Although varying depending upon the degree of deactivation, the amounts of the carbon source, nitrogen source, sulfur source and oxygen supplied, and the temperature and microorganisms, the time required for reactivation by shaking culture is usually at least 20 minutes, preferably in a range of 30 to 720 minutes. A longer activation time has not any appreciable effects. The supply of the carbon, nitrogen and sulfur sources in an amount exceeding a sufficient level gives rise to the reactivation of microorganism and, at the same time, the propagation of microorganism. For that reason, two objects, namely the activation and propagation of microorganism, are achievable in the present invention. The thus reactivated methane-utilizing bacteria can be re-used for the aforesaid oxidation reactions due to their sufficient capability of oxidizing methane.

In another embodiment of the method for regenerating the microorganisms which have lost their capability of oxidizing methane the deactivated microbial cells are supplied with oxygen and a methionine derivative during the regeneration. Preferred methionine derivatives are compounds of the following formula:

$$COOH-(CH_2)_\ell-\underset{NH_2}{\overset{H}{C}}-(CH_2)_m-S-(CH_2)_n-CH_3 \quad \ldots \quad (I)$$

wherein ℓ, m and n are 0 or 1, 1-4 and 0 or 1, respectively. The methionine derivatives of Formula (I) include e.g. methionine, β-methionine, ethionine, homomethionine and hexomethionine .

The methionine derivative may be added to the methane assimilable bacteria in a proportion of 0.02 nmol/min.•mg of cells or higher, preferably 0.05 to 50 nmol/min.•mg of cells, before or after they lose their capability of oxidizing methane. The regeneration operation may be carried out at 15 to 60 °C under aerobic conditions for 30 to 600 minutes. The methionine derivative may be continuously supplied in a certain proportion for a certain period of time, as mentioned above. Alternatively it may be added all at once in an amount corresponding to specified time at the initiation time or in the course of reactivation.

The thus regenerated methane-utilizing bacteria can be re-used for the aforesaid oxidation reaction due to their sufficient capability of oxidizing methane.

In a further embodiment of the method of the present invention, the regenerated methane-utilizing bacteria are again returned to be reactor vessel for the production of the oxides. In this manner, the oxides can be stably produced over an extended period of time. In a preferred embodiment of the present invention benzoic acid or a metal salt thereof (e.g., its alkaline metal salt or alkaline earth metal salt) is added to the reaction system (the reactor vessel and/or the regeneration vessel) so as to promote the regeneration of the deactivated microbial cells at any time during the progress of the present method, e.g., at the time of the feed of the raw material to the reactor vessel or the initiation of the reaction, or during the reaction, or at the time of the regeneration of the microbial cells. The amount of benzoic acid or its metal salt which is added is suitably in a range of 0.1 to 8 mmol/liter, preferably 0.2 to 5 mmol/liter.

In the production of the oxides, the co-oxidation capability can be increased by controlling the concentration of carbonic acid ions in the reaction solution. Concretely, carbon dioxide and/or inorganic carbonates are added to the reaction system. When carbon dioxide is used, it is preferably added as a mixed gas of air and carbon dioxide (1/0.1 to 1/0.75 by volume). Since the pH of the reaction solution drops on adding carbon dioxide, it is necessary for the carbon dioxide to be added thereto while maintaining the

pH of the reaction solution in the range of 5.5 to 9.0 by neutralizing with a basic substance such as potassium hydroxide, sodium hydroxide and ammonia. Representative examples of inorganic carbonates are potassium carbonate, sodium carbonate, sodium hydrogencarbonate and ammonium carbonate. The inorganic carbonate is added in a proportion of 1 to 140 mmol/l, preferable 1 to 130 mmol/l although the optimum amount somewhat varies with the type of the strain used.

According to the present invention, the alcohol, epoxide or cyclic alcohol corresponding to the raw material can be obtained. These products can be separated and recovered by applying known techniques such as phase separation, extraction, distillation and adsorption.

In accordance with the present invention, the desired oxides can be produced continuously and at lower costs, since the microorganisms which have lost their methane monooxygenase activity can be reactivated or regenerated for repeated use in the production process. In addition, since the present method reduces waste microorganisms and cuts down the cost of the disposal of waste liquid, it is possible to reduce the production costs of microorganisms and enzymes.

Therefore, the present invention is of significance in fields including chemical industries, pharmaceuticals, agricultural chemicals, waste water disposal and the like.

The present invention will now be explained with reference to the examples.

It is understood, however, that the cultivation of methane-utilizing bacteria was carried out by the following procedures.

8 ℓ of the medium shown in Table 1 were charged into a jar fermenter having a volume of 10 ℓ, sterilized at 120 °C for 20 minutes and, thereafter, cooled. 85 ml of the medium shown in Table 2 were sterilized at 120 °C for 20 minutes and added to the above medium.

Table 1

| | |
|---|---|
| Magnesium sulfate • $7H_2O$ | 1.0 g |
| Potassium nitrate | 1.0 g |
| Calcium chloride | 50 mg |
| $NaMoO_4$ | 1 mg |
| $FeSO_4 \cdot 7H_2O$ | 500 µg |
| $ZnSO_4 \cdot 7H_2O$ | 400 µg |
| $H_3BO_4$ | 15 µg |
| $CoCl_2 \cdot 6H_2O$ | 50 µg |
| $MnCl_2 \cdot 4H_2O$ | 20 µg |
| $NiCl_2 \cdot 6H_2O$ | 10 µg |
| $CuSO_4 \cdot 5H_2O$ | 200 µg |
| EDTA | 250 µg |
| Distilled water | 1 ℓ |

Table 2

| | |
|---|---|
| $Na_2HPO_4 \cdot 12H_2O$ | 43 g |
| $KH_2PO_4$ | 15.6 g |
| Fe-EDTA | 240 mg |
| Distilled water | 1 ℓ (pH 6.8) |

Next, 50 ml of the medium shown in Table 1 were put in each of eight Mayer's flasks having a volume of 500 ml, and sterilized at 120 °C for 20 minutes. 0.5 ml of the medium shown in Table 2 which had been sterilized at 120 °C for 20 minutes were added to each flask. This medium was inoculated with one platinum loop of methane-utilizing bacteria. After the addition of 50 ml of methane, the flask was closed with a rubber stopper and the culture was shaked for 3 days at 30 to 45 °C. The culture solutions were then sterily transferred as the seed culture from the eight flasks into the aforesaid jar fermenter, wherein a culture was carried out for 3 days, while supplying thereto a gaseous mixture of methane and air (methane : air = 1 : 4) in a rate of 4 ℓ per minute. After the concentration of the microbial cells had reached 1.5 mg/ml, a medium obtained by adding $CuSO_4 \cdot 5H_2O$ in a proportion of 1 mg/l to a medium prepared by mixing together the media of Tables 1 and 2 in a proportion of 100 : 1.5 was supplied to the jar fermenter in a rate of 1.6 ℓ/hour

for continuous culture, while passing through a sterile filter.

Examples 1 to 5 and Comparative Example 1

2 ℓ of a culture solution of Methylococcus capsulatus NCIB 11132 continuously cultured according to the culture method of methane-utilizing bacteria were centrifuged to collect the microorganisms.

The collected microorganisms were suspended in the reactivation solution shown in Table 3 at a regulated cell concentration of 3 mg/ml. Four hundred (400) ml of this suspension were charged into a jar fermenter of 1 liter volume. The temperature was then increased to 45 °C. While passing air through the jar fermenter at a rate 200 ml per minute, methanol was added to the suspension at once at a regulated concentration of 10 mM. In addition, acetylene that was a killer substrate for the methane oxidation enzymes of methane-utilizing bacteria was supplied for 1 minute at a rate of 2 ml per minute to deactivate the capability of oxidizing methane thereof. Ten minutes later, the activity of the microorganisms in the reaction solution was measured. Afterwards, while supplying air at a rate of 200 ml per minute, methanol was supplied under agitation at 900 r.p.m. to this solution at the rates shown in Table 4. 240 minutes later, the activity of the microorganisms in the jar fermenter was measured. During reactivation, the culture solution was maintained at pH 7 with 1M nitric acid and 1M caustic potash. The results are set forth in Table 4.

Table 3

| (Reactivation Solution) | |
|---|---|
| Magnesium sulfate • $7H_2O$ | 1.0 g |
| Potassium nitrate | 1.0 g |
| Calcium chloride | 100 mg |
| $NaMoO_4$ | 1 mg |
| $FeSO_4 \cdot 7H_2O$ | 500 μg |
| $ZnSO_4 \cdot 7H_2O$ | 400 μg |
| $H_3BO_4$ | 15 μg |
| $CoC\ell_2 \cdot 6H_2O$ | 50 μg |
| $MnC\ell_2 \cdot 4H_2O$ | 20 μg |
| $NiC\ell_2 \cdot 6H_2O$ | 10 μg |
| $CuSO_4 \cdot 5H_2O$ | 1500 μg |
| EDTA | 250 μg |
| $Na_2HPO_4 \cdot 12H_2O$ | 645 mg |
| $KH_2PO_4$ | 234 mg |
| Fe-EDTA | 3.6 mg |
| Distilled water | 1 ℓ (pH 6.8) |

**Table 4**

| | Methanol Feeding Rate (nmol/min.·mg of microorganism) | Activity of Microorganism (nmol/min.·mg of microorganism) I | II | III |
|---|---|---|---|---|
| Ex.1 | 30 | 401 | 7 | 286 |
| 2 | 50 | 389 | 4 | 342 |

Table 4 (continued)

| | Methanol Feeding Rate (nmol/min.·mg of microorganism) | Activity of Microorganism (nmol/min.·mg of microorganism) I | II | III |
|---|---|---|---|---|
| Ex. 3 | 80 | 401 | 6 | 408 |
| 4 | 200 | 378 | 4 | 380 |
| 5 | 400 | 393 | 6 | 378 |
| Comp. Ex. 1 | 0 | 387 | 7 | 32 |

I : before acetylene addition

II : 10 minutes after acetylene addition

III : 240 minutes after methanol feeding

As clearly understood from Table 4, satisfactory results are obtained, when methanol is supplied at a rate of 10 nmol/min.•mg of microorganism or higher, while any appreciable effect is not obtained, when methanol is supplied at a rate of higher than 400 nmol/min.•mg of microorganism, which brings about an increase in the production cost.

Incidentally, the activity of microorganisms was measured by the following procedures. The microbial cells were suspended in 5 mM of a PIPS buffer at a regulated concentration of 0.5 mg/mℓ, and 1 mℓ of the suspension was put in a Mayer's flask of 7 mℓ in volume, to which 2 mℓ of propylene were added. After the flask had been closed with a rubber plug, the culture incubated at 45°C for 30 seconds. Then, methanol was added at a regulated concentration of 1 mM for a culture of further 3 minutes. Afterwards, the amount of propylene oxide produced was determined by chromatography, and was expressed in terms of the amount of propylene oxide produced per mg of microbial cells per minute.

Examples 6 to 9 and Comparative Example 2

A culture was carried out in a manner analogous to that described in Example 1, except that Methylococcus capsulatus NCIB 11132 continuously cultured according to the culture method of methane-utilizing bacteria was applied at a regulated biocell concentration of 0.5 mg/ml. Afterwards, the methane oxidation activity of the microorganism was deactivated with acetylene. Ten minutes later, 10 ml of the culture solution were transferred from the jar fermenter into a Mayer's flask of 100 ml in volume, to which methanol was added at the concentrations specified in Table 5. That flask was closed with a cotton plug, and shaken at 45°C and 200 r.p.m. 240 minutes later, the activity of the microorganism was measured. The results are set forth in Table 5. This activity was 346 nmol/min.•mg of microorganism and 0 nmol/min.•mg of microorganism before and 10 minutes after the addition of actylene, respectively.

Table 5

| | Concentration of Methanol (mM) | Activity of Microorganism in 240 min. After Acetylene Addition (nmol/min.•mg of microorganism) |
|---|---|---|
| Ex. 6 | 6 | 230 |
| 7 | 12 | 344 |
| 8 | 18 | 367 |
| 9 | 24 | 382 |
| Comp. Ex. 2 | 0 | 28 |

Examples 10 to 15 and Comparative Example 3

Example 1 was repeated, except that acetylene was supplied at a rate of 0.5 ml/min. for 1 minute and ten minutes later a mixed gas of methane and air was supplied at a rate of 200 ml/min. in place of methanol. The results are set forth in Table 6.

Table 6

| | Methane/Air Ratio (%) of Mixed Methane/Air Gas | | Activity of Microorganism (nmol/min.•mg of microorganism) | | |
|---|---|---|---|---|---|
| | | | I | II | III |
| Ex. 10 | 3 | 97 | 431 | 24 | 474 |
| 11 | 6 | 94 | 428 | 17 | 472 |
| 12 | 77 | 23 | 451 | 26 | 478 |
| 13 | 83 | 17 | 444 | 27 | 460 |
| 14 | 91 | 9 | 444 | 25 | 294 |
| 15 | 97 | 3 | 456 | 23 | 182 |
| Comp. Ex. 3 | 100 | 0 | 429 | 24 | 21 |

I: before acetylene addition
II: 10 minutes after acetylene addition
III: 240 minutes after the feed of mixed gas of methane and air

Any special limitation is not imposed upon the ratio of methane to air. As clearly understood from Table 6, however, the reactivation of methane-utilizing bacteria is retarded in a state extremely short of oxygen, and does not occur at all in the absence of oxygen.

Examples 16 to 20

Example 1 was repeated, except that formaldehyde was supplied in place of methanol at the rates specified in Table 7 and 140 minutes later the activity of the microorganism was measured. The results are set forth in Table 7.

Table 7

| | Formaldehyde Feed Rate (nmol/min.•mg of microorganism) | Activity of Microorganism (nmol/min.•mg of microorganism) | | |
|---|---|---|---|---|
| | | I | II | III |
| Ex. 16 | 30 | 376 | 4 | 173 |
| 17 | 50 | 398 | 0 | 205 |
| 18 | 100 | 398 | 8 | 210 |
| 19 | 200 | 378 | 0 | 180 |
| 20 | 400 | 392 | 7 | 136 |

I : before acetylene addition
II : 10 minutes after acetylene addition
III : 240 minutes after formaldehyde feed

From Table 7, it is clear that difficulties are encountered in the reactivation of the methane-utilizing bacteria due to the toxicity of formaldehyde, when formaldehyde is supplied at a rate of 400 nmol/min.•mg of microorganism or higher.

Comparative Example 4

Example 18 was repeated, except that potassium formate was supplied at a rate of 100 nmol/min.•mg of microbial cells in place of formaldehyde. As a result, the activity of the microbial cells was 387 nmol/min.•mg of microbial cells before the addition of acetylene, 8 nmol/min.•mg of microbial cells 10 minutes after the addition of acetylene and 24 nmol/min.•mg of microbial cells 140 minutes after the supply of potassium formate.

Examples 21 to 24

Example 3 was repeated, except that the nitrogen source shown in Table 8 was substituted for potassium nitrate in the reactivation solution shown in Table 3, and the adjustment of pH during reactivation was performed with 1 M hydrochloric acid and 1 M caustic potash. The results are set forth in Table 8.

Table 8

| | Nitrogen Source (mg/ℓ) | | Activity of Microorganism (nmol/min.•mg of microorganism) | | |
|---|---|---|---|---|---|
| | | | I | II | III |
| Ex. 21 | Ammonium Sulfate | 570 | 372 | 7 | 286 |
| 22 | Peptone | 500 | 366 | 9 | 342 |
| 23 | L-glutamine | 200 | 367 | 7 | 378 |
| 24 | L-asparagine | 200 | 378 | 8 | 354 |

I : before addition of acetylene
II : 10 minutes after addition of acetylene
III : 240 minutes after methanol feed

Comparative Example 5

Example 3 was repeated, except that potassium chloride was substituted for potassium nitrate in the reactivation solution shown in Table 3, and the adjustment of pH during reactivation was performed with potassium hydroxide. The activity of the microorganisms was 372 nmol/min.•mg of microorganism before the addition of acetylene, 9 nmol/min.•mg of microorganism 10 minutes after the addition of acetylene and 24 nmol/min.•mg of microorganism 240 minutes after the feeding of methanol.

Examples 25 to 28 and Comparative Example 6

A culture solution of Methylococcus capsulatus NCIB 11132 cultured according to the culture method of methane-utilizing bacteria was centrifuged and washed three times with the reactivation solution shown in Table 9 which contained the salts shown in Table 10. Thereafter, the microbial cells were suspended in the same reactivation solution at a regulated concentration of microbial cells of 3 mg/mℓ.

Table 9

| (Reactivation Solution) | |
|---|---|
| Potassium nitrate | 1.0 g |
| Calcium chloride | 100 mg |
| Magnesium chloride | 200 mg |
| $Na_2HPO_4 \cdot 12H_2O$ | 645 mg |
| $KH_2PO_4$ | 234 mg |
| Distilled water | 1ℓ (pH 6.8) |

400 mℓ of this suspension were charged into a jar fermenter of 400 mℓ in volume, the temperature of which was then increased to 45 °C. After propylene oxide had been added to the suspension in a proportion of 2 nmol/400 mℓ, air and methanol were supplied thereto in the respective proportions of 80 mℓ/min. and 300 nmol/min.•mg of microbial cells for 30 minutes. 30 minutes later, the microbial cells were centrifuged out for the removal of the remaining propylene oxide, and were again suspended in the same reactivation solution. 50 minutes later, methanol and air were supplied to the suspension in the respective proportions of 80 nmol/min•mg of microorganism and 40 mℓ/min, and the sulfur compounds shown in Table 10 were added thereto. The suspension was stirred for further 240 minutes. The results are set forth in Table 10.

Table 10

| | Sulfur Source (mg/ℓ) | | Activity of Microorganism (nmol/min.•mg of microorganism) | | |
|---|---|---|---|---|---|
| | | | I | II | III |
| Ex. 25 | Sodium sulfate | 50 | 403 | 24 | 376 |
| 26 | Sodium sulfide | 50 | 387 | 32 | 362 |
| 27 | Hydrosulfide | 50 | 387 | 36 | 343 |
| 28 | Sodium sulfhydrate | 50 | 392 | 18 | 346 |
| Comp. Ex. 6 | Not added | | 380 | 32 | 24 |

I : before addition of propylene oxide
II : 30 minutes after addition of propylene oxide
III : 240 minutes after feeding of methanol and sulfur

Example 29

A culture solution of Methylococcus capsulatus NCIB 11132 continuously cultured according to the culture method of methane-utilizing bacteria was diluted with the reactivation solution shown in Table 3 to a regulated microorganism concentration of 3 mg/ml. 400 ml of the thus diluted solution were charged in a jar fermenter of 1 ℓ in volume, the temperature of which was then increased to 45 °C. That solution was stirred at 900 r.p.m., while passing propylene and air at the respective rates of 150 ml and 50 ml per minute. Simultaneously with the supply of propylene, methanol was supplied in a proportion of 300 nmol/mg of microorganism per minute. 90 minutes later, the feed of propylene and methanol was interrupted, and propylene oxide accumulated in the reaction solution was expelled by the supply of air at a rate of 3.2 ℓ per minute. 20 minutes later, the supply of air was interrupted, and a mixed gas of methane and air (methane : air = 4 : 1) was supplied at a rate of 200 ml per minute.

240 minutes later, the activity of the microorganism in the jar fermenter was measured. It is to be noted that during reactivation, the microorganism did not propagate. The results are set forth in Table 11.

Table 11

| Amount of Propylene Oxide in Water in 90 minutes After Propylene Feeding | Activity of Microorganism (nmol/min.•mg of microorganism) | | |
|---|---|---|---|
| | I | II | III |
| 2.6 | 380 | 178 | 355 |
| I : before addition of propylene<br>II : 90 minutes after addition of propylene<br>III : 240 minutes after feeding of mixed gas of methane and air | | | |

Example 30

A culture solution of Methylococcus capsulatus NCIB 11132 continuously cultured according to the culture method of methane-utilizing bacteria was diluted with the reactivation solution shown in Table 3 to a regulated microbial cells concentration of 3 mg/mℓ.
400 mℓ of the thus diluted solution were charged in a jar fermenter of 1 ℓ in volume, the temperature of which was then increased to 37.5°C. That solution was stirred at 900 r.p.m., while passing 1-butene and air at the respective rates of 320 mℓ and 80 mℓ per minute. Simultaneously with the supply of 1-butene, methanol was supplied in a proportion of 300 nmol/mg of microbial cells per minute. After 150 minutes, the supply of 1-butene and methanol was interrupted. The reaction solution was centrifuged for the removal of 1,2-butylene oxide, and was again suspended in 400 mℓ of a fresh reactivation solution as shown in Table 3. In the meantime, the time required for centrifugation and resuspension was 20 minutes. Then, the resulting solution was maintained at 37.5°C, while supplying thereto air and methane in the respective rates of 40 mℓ and 160 mℓ. After 400 minutes, the activity of the microorganism in the jar fermenter was measured. It is to be noted that during reactivation, the microorganism did not propagate. The results are set forth in Table 12.

Table 12

| Amount of Butylene Oxide in Water in 150 min. After 1-Butene Supply (mM) | Activity of Microorganism (nmol/min.•mg of microorganism) | | |
|---|---|---|---|
| | I | II | III |
| 6.2 | 332 | 106 | 354 |
| I : before addition of 1-butene<br>II : 150 minutes after addition of 1-butene<br>III : 400 minutes after feeding of air and methane | | | |

Examples 31 and 34

Four Mayer's flasks of 500 mℓ in volume, each containing 50 mℓ of a medium prepared by the method of Whittenbury et al (J. Gen. Microbiol., 61, pp. 205-208, 1970), were prepared and sterilized under pressure at 120°C for 15 minutes. After cooling, the gaseous phases were replaced with a mixed gas of methane and air having a methane/air ratio of 1 : 4. Then, cells of methylomonas agile NCIB 11124, Methylocystis parvus NCIB 11129, Methylosinus trichosporium NCIB 11131 and Methylobacter capsulata NCIB 11128 were inoculated into the media and the culture was shaken at 30°C for 32 hours.

After the completion of the culture, the culture solutions were centrifuged to collect the microorganism, which were in turn suspended in the reactivation solution specified in Table 13 at a regulated microorganism concentration of 1 mg/mℓ. 10 mℓ of this suspension were put in a Mayer's flask of 250 mℓ in volume, which was closed with a rubber stopper after addition of 2 mM of methanol and 100 µℓ acetylene. After the shaken culture had been carried out at 30°C for 10 minutes, the rubber stopper was pulled out of the flask

to pass air therethrough at a rate of 1 ℓ per minute for 10 minutes, thereby removing the remaining acetylene. 100 mℓ of methane were added to the flask, which was then closed with the rubber stopper for shaking at 30 °C for 220 minutes. Thereafter, the activity of the microorganism was measured. The results are set forth in Table 14.

Table 13 (Reactivation Solution)

| | |
|---|---|
| Magnesium sulfate·$7H_2O$ | 1.0 g |
| Potassium nitrate | 1.0 g |
| Calcium chloride | 100 mg |
| $Na_2HPO_4 \cdot 12H_2O$ | 645 mg |
| $KH_2PO_4$ | 234 mg |
| Distilled water | 1 ℓ |

Table 13 (Reactivation Solution) (contn'd)

Table 14

| | Bacteria | Activity of Microorganism (nmol/min.•mg of microorganism) | | |
|---|---|---|---|---|
| | | I | II | III |
| Ex. 31 | Methylomonas agile NCIB 11124 | 82 | 0 | 64 |
| Ex. 32 | Methylocystis parvus NCIB 11129 | 44 | 0 | 32 |
| Ex. 33 | Methylosinus trichosporium NCIB 11131 | 92 | 0 | 78 |
| Ex. 34 | Methylobacter capslata NCIB 11128 | 72 | 0 | 81 |

I : before acetylene addition
II : 10 minutes after acetylene addition
III : 240 minutes after air feeding

Examples 35 to 41

400 mℓ of each culture solution of Methylomonas methanica NCIB 11130, Methylomonas agile NCIB 11124, Methylomonas albus NCIB 1112, Methylosinus trichosporium NCIB 11131, Methylosinus sporium NCIB 11126, Methylocystis parvus NCIB 11129 and Methylobacter capsulata NCIB 11128 were centrifuged to collect the microbial cells. The collected microbial cells were suspended in the reactivation solution shown in Table 15 at a regulated concentration of microbial cells of 0.5 mg/mℓ. 400 mℓ of this suspension were charged in a jar fermenter of 1 ℓ in volume, the temperature of which was increased to 31 °C. Then, this suspension was stirred at 900 r.p.m., while passing propylene and air through the jar fermenter at the respective rates of 150 mℓ and 150 mℓ per minute. Simultaneously with the supply of propylene, methanol was supplied in a rate of 300 nmol/mg of microbial cells per minute.

30 minutes later, the supply of propylene and methanol was interrupted, and air was supplied at a rate of 5 ℓ per minute to expel propylene oxide accumulated in the reaction solution. 20 minutes later, the amount of air supplied was decreased to 25 mℓ, and methane was supplied in a further rate of 110 mℓ/min.

180 minutes and 300 minutes after the supply of a mixed methane/air gas, the activity of the microorganism in the jar fermenter was measured. The results are set forth in Table 15.

Table 15

| (Reactivation Solution) | |
|---|---|
| Magnesium sulfate•$7H_2O$ | 1.0 g |
| Potassium nitrate | 1.0 g |
| Calcium chloride | 50 mg |
| $NaMoO_4$ | 1 mg |
| $FeSO_4 \cdot 7H_2O$ | 500 μg |
| $ZnSO_4 \cdot 7H_2O$ | 400 μg |
| $H_3BO_4$ | 15 μg |
| $CoCl_2 \cdot 6H_2O$ | 50 μg |
| $MnCl_2 \cdot 4H_2O$ | 20 μg |
| $NiCl_2 \cdot 6H_2O$ | 10 μg |
| $CuSO_4 \cdot 5H_2O$ | 500 μg |
| EDTA | 250 μg |
| $Na_2HPO_4 \cdot 12H_2O$ | 645 mg |
| $KH_2PO_4$ | 234 mg |
| Fe-EDTA | 3.6 mg |
| Distilled water | 1 ℓ (pH 6.8) |

Table 16

| Example No. | Bacteria | Amount of Propylene Oxide in Water 30 min After Propylene Supply (mM) | Activity of Microorganism (nmol/min.·mg of microorganism) | | | |
|---|---|---|---|---|---|---|
| | | | Before Propylene Supply | 30 min. After Propylene Supply | After the Supply of Mixed Methane/Air Gas | |
| | | | | | 180 min. | 300 min. |
| 35 | Methylomonas methanica NCIB 11130 | 1.9 | 472 | 108 | 247 | 338 |
| 36 | Methylomonas agile NCIB 11124 | 1.7 | 402 | 83 | 104 | 152 |
| 37 | Methylomonas albus NCIB 11123 | 1.9 | 382 | 79 | 205 | 314 |
| 38 | Methylosinus trichosporium NCIB 11131 | 2.7 | 372 | 116 | 398 | 409 |
| 39 | Methylosinum sporium NCIB 11126 | 1.8 | 402 | 148 | 332 | 401 |
| 40 | Methylocystis parvus NCIB 11129 | 2.3 | 490 | 112 | 410 | 506 |
| 41 | Methylobacter capsulata NCIB 11128 | 1.4 | 332 | 134 | 259 | 346 |

Examples 42 to 47 and Comparative Example 7

The reactivation solution specified in Table 15 was added to a culture solution of Methylococcus capsulatus NCIB 11132 continuously cultured at 45 °C according to the culture method of methane-utilizing bacteria in such a manner that the microorganism concentration was in the order of 0.3 mg/l, and 500 ml of

the resulting solution was then charged in a jar fermenter of 1 ℓ in volume, the temperature of which was in turn increased to 45 °C. Thereafter, while passing 110 ml/min of methane and 50 ml/min. of air through that fermenter, 1 ml of acetylene was added thereto at once to deactivate the capability of oxidizing methane of the microorganism .

10 minutes later, the microorganisms were centrifuged and washed three times with the reactivation solution specified in Table 17. Thereafter, the microorganisms were suspended in the same reactivation solution at a regulated microorganism concentration of 1.45 mg/ml. Afterwards, 10 ml portions of the suspension were put in Mayer's flasks of 250 ml in volume.

Then, varied concentrations of potassium nitrate were added into such flasks, followed by closing with stoppers. Thereafter, 50 ml portions of methane were added into the flasks for 80 r.p.m. shaking culture at 45 °C.

3 hours later, the activity of the microorganism was measured the results are set forth in Table 18.

Table 17

| | |
|---|---|
| Magnesium sulfate•$7H_2O$ | 1 g |
| Magnesium chloride•$6H_2O$ | 203 mg |
| Calcium chloride | 50 mg |
| $NaMoO_4$ | 1 mg |
| $FeCl_3$•$6H_2O$ | 500 μg |
| $ZnCl_2$ | 200 μg |
| $H_3BO_4$ | 15 μg |
| $CoCl_2$•$6H_2O$ | 50 μg |
| $MnCl_2$•$4H_2O$ | 20 μg |
| $NiCl_2$•$6H_2O$ | 10 μg |
| $CuCl_2$•$2H_2O$ | 340 μg |
| EDTA | 250 μg |
| $Na_2HPO_4$•$12H_2O$ | 645 mg |
| $KH_2PO_4$ | 234 mg |
| Fe-EDTA | 3.6 mg |
| Distilled water | 1 ℓ |

Table 18

| | Amount of Potassium Nitrate Added (g/ℓ) | Activity of Microorganism (nmol/min.·mg microorganism) I | II |
|---|---|---|---|
| Ex. 42 | 0.026 | 0 | 105 |
| 43 | 0.065 | 0 | 133 |

Table 18 (contn'd)

| | Amount of Potassium Nitrate Added (g/ℓ) | Activity of Microorganism (nmol/min.•mg microorganism) | |
|---|---|---|---|
| | | I | II |
| Ex. 44 | 0.13 | 0 | 180 |
| 45 | 0.26 | 0 | 307 |
| 46 | 0.79 | 0 | 401 |
| 47 | 2.63 | 0 | 337 |
| Comp. Ex. 7 | 0 | 0 | 85 |

I : 10 min. after addition of acetylene

II : 180 min. after addition of air and methane

It is noted that the activity of the microorganism was 578 nmol/min.•mg of microorganism before the addition of acetylene.

Examples 48 to 53 and Comparative Example 8

Experiments were conducted in a manner similar to that described in Examples 42 to 47, except that 1 g/ℓ of potassium nitrate was added in place of magnesium sulfate•$7H_2O$shown in Table 17, and varied concentrations of magnesium sulfate•$7H_2O$ were added in place of potassium nitrate shown in Table 18. The results are set forth in Table 19.

Table 19

| | Amount of Magnesium Sulfate•$7H_2O$ (g/ℓ) | Added Activity of Microorganism (nmol/min.•mg microorganism) | |
|---|---|---|---|
| | | I | II |
| Ex. 48 | 0.013 | 0 | 229 |
| 49 | 0.063 | 0 | 257 |
| 50 | 0.013 | 0 | 362 |
| 51 | 0.032 | 0 | 402 |
| 52 | 0.064 | 0 | 424 |
| 53 | 0.32 | 0 | 295 |
| Comp. Ex. 8 | 0 | 0 | 183 |

I : 10 minutes after addition of acetylene

II : 180 minutes after the feed of air and methane

Examples 54 to 59 and Comparative Example 9

In the culture method of methane assimilable bacteria, the concentration of magnesium sulfate•$7H_2O$ in a medium was adjusted to 7 g/ℓ. Methylocystis parvus NCIB 11129 was cultured at 31 °C.

500 mℓ of a microbial culture solution (having a microorganism concentration of 0.49 mg/mℓ) were placed in a 1 liter-jar fermenter, the temperature of which was then increased to 33 °C. Thereafter, while supplying 150 mℓ/min. of propylene and 150 mℓ/min. of air to the jar fermenter, that solution was stirred at 900 r.p.m. Simultaneously with the supply of propylene, methanol was supplied at a rate of 300 nmol/mg of microorganism per minute. 30 minutes later, the supply of propylene and methanol was interrupted, and air was supplied at a rate of 5 ℓ per minute to expel propylene oxide accumulated in the reaction solution. Subsequently, the microorganisms were centrifuged and washed four times with the reactivation solution shown in Table 17, from which magnesium sulfate•$7H_2O$had been removed. Thereafter, the microorganisms were suspended in the same solution at a microorganism concentration regulated to 0.5 mg/mℓ, and 20 mℓ portions of the suspension were placed in Mayer's flasks of 250 mℓ in volume. Varied concentrations of magnesium sulfate•$7H_2O$ were added into the flasks, followed by closing with rubber plugs. Thereafter, 50 mℓ of methane were added to the flasks, which were then shaken at 30 °C and 80 r.p.m.

300 minutes after the initiation of the shaking culture, the activity of the microorganisms was measured. The results are set forth in Table 20.

Table 20

| | Amount of Magnesium Sulfate $7H_2O$ Added (g/ℓ) | Activity of Microorganism (nmol/min.·mg of microorganism) 300 min. After the Addition of Mixed Methane/Air Gas |
|---|---|---|
| Ex. 54 | 1.0 | 432 |
| 55 | 3.5 | 428 |

Table 20 (contn'd)

| | Amount of Magnesium Sulfate $7H_2O$ Added (g/ℓ) | Activity of Microorganism (nmol/min.·mg of microorganism) 300 min. After the Addition of Mixed Methane/Air Gas |
|---|---|---|
| Ex. 56 | 7.0 | 403 |
| 57 | 16.0 | 461 |
| 58 | 30.0 | 326 |
| 59 | 40.0 | 282 |
| Comp. Ex. 9 | 0 | 68 |

It is noted that propylene oxide in water was 2.2 mM at 30 minutes after the supply of propylene, the activity of the microorganism before the supply of propylene was 449 nmol/min.•mg of microorganism and the activity of the microorganism after centrifugation and washing was 142 nmol/min.•mg of microorganism.

Examples 60-63 and Comparative Example 10

2 ℓ of a culture solution of Methylococcus capsulatus NCIB 11132 continuously cultured according to the culture method of methane-utilizing bacteria were centrifuged to collect microbial cells, which were thereafter suspended in 200 ml of a 4-mM phosphate buffer (pH 6.8), and the resulting suspension was again centrifuged to wash the microbial cells. Such washing was repeated two times.

Afterwards, the microbial cells were suspended in a 4-mM phosphate buffer (pH 6.8) at a bacterial concentration of 2 mg/ml, and 400 ml of the resulting suspension were put in a jar fermenter of 1 ℓ in volume, which was in turn elevated to a temperature of 45°C. Then, while air was supplied to the jar fermenter at a rate of 40 ml per minutes, potassium formate was added thereto at a concentration of 10 mM. Further, acetylene that is a killer substrate for methane monooxygenase was supplied to the jar fermenter at a rate of 1 ml per minute for 1 minute to deactivate the methane oxidizability. After 10 minutes, L-methionic acid was added to the jar fermenter at the rates specified in Table 21, and the activity of the microbial cells therein was determined after 240 minutes. It is noted that during the regeneration of the microbial cells, the solution of microbial cells was maintained at pH 7.0 with formic acid. The results are set out in Table 21.

Table 21

| | Amount of L-Methionine (mg/ℓ) | Activity of Microbial Cells (nmol/min.•mg of cells) | | |
|---|---|---|---|---|
| | | Before Acetylene Addition | 10 min. After Acetylene Addition | 240 min. After L-methionine Addition |
| Ex. 60 | 10 | 316 | 0 | 182 |
| 61 | 25 | 298 | 0 | 206 |
| 62 | 50 | 308 | 0 | 212 |
| 63 | 100 | 288 | 0 | 176 |
| Comp. Ex. 10 | 0 | 298 | 0 | 7 |

Example 64 and Comparative Example 11

Methylococcus capsulatus NCIB 11132 cultured according to the culture method of methane-utilizing bacteria was collected, suspended and cultured in a similar manner as described in Example 60, and propylene oxide in place of acetylene was added all at once at a concentration of 4 mM to deactivate the methane oxidizability. After 30 minutes, air was supplied at a rate of 3.2 ℓ per minute for 10 minutes to remove propylene oxide, and 50 mg/l of L-methionine were added, while supplying air at a rate of 40 m/min. After 240 minutes, the activity of microbial cells was measured. It is noted that Comparative Example 11 was carried out in a similar manner as in Example 64, but in the absence of L-methionine. The results are set out in Table 22.

Table 22

| | Amount of L-Methionine (mg/ℓ) | Activity of Microbial Cells (nmol/min.•mg of cells) | | |
|---|---|---|---|---|
| | | Before Propylene Oxide Addition | 10 min. After Propylene Oxide Addition | 240 min. After L-methionine Addition |
| Ex. 64 | 50 | 274 | 26 | 202 |
| Comp. Ex. 11 | 0 | 280 | 31 | 16 |

Examples 65 to 70 and Comparative Examples 12 to 14

200 ml of a culture solution of Methylococcus capsulatus NCIB 11132 continuously cultured according to the culture method of methane-utilizing bacteria were centrifuged to collect the microorganisms, which were then suspended in the regeneration solution specified in Table 23 at a regulated concentration of microbial cells of 1 mg/ml.

400 ml of this suspension were charged into a jar fermenter of 1 liter in volume, the temperature of which was in turn increased to 45°C, while supplying air thereto at a rate of 100 ml per minute. Subsequently, sodium benzoate was added to that fermenter in the amount specified in Table 24, followed by 10 minute-stirring. Afterwards, propylene and methanol were supplied to that fermenter for 30 minutes at the respective rates of 320 ml/min. and 350 nmol/min.•mg of microorganism to produce propylene oxide.

After 30 minutes, the supply of propylene and methanol was interrupted, and air was supplied at a rate of 3.2 liters per minute to expel the remaining propylene oxide out of the reaction solution. After 15 minutes, the rate of the air supplied was decreased to 35 ml/min. After the temperature had been identified to be 45°C, the regeneration of the microorganism was initiated by the supply of methane at a rate of 120 ml/min. Thereafter, the activity of the microorganism was measured in succession to determine the time by which the regeneration of the microorganism occurred. The results are set forth in Table 24.

Table 23

| (Regeneration Solution) | |
|---|---|
| Magnesium sulfate•$7H_2O$ | 1.0 g |
| Potassium nitrate | 1.0 g |
| Calcium chloride | 100 mg |
| $NaMoO_4$ | 1 mg |
| $FeSO_4 \cdot 7H_2O$ | 500 µg |
| $ZnSO_4 \cdot 7H_2O$ | 400 µg |
| $H_3BO_4$ | 15 µg |
| $CoCl_2 \cdot 6H_2O$ | 50 µg |
| $MnCl_2 \cdot 4H_2O$ | 20 µg |
| $NiCl_2 \cdot 6H_2O$ | 10 µg |
| $CuSO_4 \cdot 5H_2O$ | 500 µg |
| EDTA | 250 µg |
| $Na_2HPO_4 \cdot 12H_2O$ | 645 mg |
| $KH_2PO_4$ | 234 mg |
| Fe-EDTA | 3.6 mg |
| Distilled water | 1 liter (pH 6.8) |

Table 24

| | Amount of Sodium Benzoate Added (mM) | Microbial Activity at the Time of Initiation of Reaction (nmol/min·mg microorganism) | Microbial Activity at the Time of Regeneration (nmol/min·mg microorganism) | Time until Initiation of Regeneration (min.) |
|---|---|---|---|---|
| Example 65 | 0.25 | 390 | 67 | 105 |
| " 66 | 0.88 | 386 | 64 | 95 |
| " 67 | 1.75 | 388 | 54 | 85 |
| " 68 | 2.50 | 380 | 58 | 50 |
| " 69 | 3.75 | 386 | 62 | 120 |
| " 70 | 5.0 | 490 | 56 | 115 |
| Comparative Example 12 | 10.0 | 487 | 58 | 175 |
| " 13 | 20.0 | 398 | 60 | 190 |
| " 14 | 0 | 496 | 57 | 180 |

It is noted that the activity of microorganisms was measured in the following manner. The microorganisms were suspended in a 5 mM PIPES buffer at a regulated concentration of 0.5 mg/ml, and 1 ml of the suspension was put in a Mayer's flask of 7 ml in volume. The flask was closed with a rubber stopper, followed by the addition of 2 ml of propylene, and was then shaken with a rate of 300 r.p.m. at 45 °C for 30 seconds to agitate the content. Subsequently, methanol was added to the flask at a regulated concentration

of 1 mM, which was cultured under shaking for 3 minutes. Afterwards, gas chromatography was applied to determine the amount of the propylene oxide produced per minute per 1 mg of microorganism.

Examples 71 to 74 and Comparative Examples 15, 16

Operations similar to those described in Example 65 were carried out, except that 1-butene was substituted for propylene, and air was supplied at a rate of 4 liters per minute for the removal of butylene oxide after the termination of the reaction. The results are set forth in Table 25.

Table 25

| | Amount of Sodium Benzoate Added (mM) | Microbial Activity at the Time of Initiation of Reaction (nmol/min·mg microorganism) | Microbial Activity at the Time of Regeneration (nmol/min·mg microorganism) | Time until Initiation of Regeneration (min.) |
|---|---|---|---|---|
| Example 71 | 0.25 | 372 | 92 | 40 |
| " 72 | 0.88 | 381 | 88 | <30 |
| " 73 | 2.50 | 378 | 83 | <30 |
| " 74 | 5.0 | 372 | 85 | 60 |
| Comparative Example 15 | 10.0 | 381 | 81 | 125 |
| " 16 | 0 | 382 | 93 | 120 |

Examples 75 to 77 and Comparative Examples 17, 18

Operations similar to those described in Example 65 were carried out, except that benzoic acid was used in place of sodium benzoate, and adjusted pH to 7.0 with 0.5M potassium hydroxide. The results are

shown in Table 26.

Table 26

| | Amount of Benzoic Acid Added (mM) | Microbial Activity at the Time of Initiation of Reaction (nmol/min·mg microorganism) | Microbial Activity at the Time of Regeneration (nmol/min·mg microorganism) | Time until Initiation of Regeneration (min.) |
|---|---|---|---|---|
| Example 75 | 0.25 | 418 | 71 | 100 |
| " 76 | 1.75 | 426 | 68 | 75 |
| " 77 | 5.0 | 440 | 64 | 110 |
| Comparative Example 17 | 0 | 389 | 58 | 185 |
| " 18 | 10.0 | 443 | 59 | 180 |

Example 78

This example was carried out with using the apparatus system shown in Figure 1.

2.4 liters of a medium obtained by mixing together the medium specified in Table 1 except that the concentration of $CuSO_4 \cdot 5H_2O$ was changed to 1000 µg and the medium specified in Table 2 in a mixing ratio of 100 : 1.5 were charged into a regenerating vessel B through a sterile filter.

On the other hand, 50 ml portions of a medium having the same composition, which had been treated through a sterile filter for the removal of infectious microbes, were charged into six Mayer's flasks of 500 ml in volume. One platinum loop of slant-cultured Methylococcus capsulatus NCIB 11132 was inoculated into each flask, which was then closed with a rubber plug, followed by pouring of 100 ml of methane. Culture was subsequently carried out at 45°C for 3 days. The obtained culture solution amounting to 300 ml in all was inoculated on the medium contained in the vessel B described above. While supplying a mixed methane/air gas of 1 : 4 at a rate of 1.35 liters per minute to the regeneration vessel B maintained at 45°C, culture was carried out at 600 r.p.m. for 24 hours. In consequence, the microbial concentration reached to 1 mg/ml.

Next, the microbial suspension was supplied to a reactor vessel A at a rate of 20 ml per minute by means of a pump 1. At the same time, a pump 2 was started to feed the microbial suspension from the reactor A to the upper portion of a scrubber C at a rate of 60 ml per minute. It is here noted that the reactor and scrubber were maintained at 45°C with hot water.

Simultaneously with air supply at a rate of 120 ml per minute, a 10 v/v % methanol solution was supplied under 900 r.p.m. agitation to the reactor vessel at a rate of 65 µl per minute. A pump 3 was started to guide the microbial suspension from the reactor vessel to the upper portion of a scrubber D at a rate of 20 ml per minute and return it to the regenerating vessel through its lower portion. All the amount of the mixed methane/air effluent gas was admitted into the lower portion of the scrubber D. A gas leaving the upper portion of the scrubber D is guided into the lower portion of the scrubber C, and was vented from the upper portion thereof. After the apparatus system had been operated for 24 hours in such a state, the overall microbial concentration in the reaction system reached 2.2 mg/ml.

A 10 v/v % methanol solution was supplied to the regenerating vessel at a rate of 100 µl/min. and, at the same time, the amount of the methanol supplied to the reactor vessel was decreased to 25 µl/min. Subsequently, propylene was fed from the lower portion of the reactor vessel at a rate of 320 ml/min to initiate the production of propylene oxide. More exactly, the microorganism-containing reaction mixture was supplied from the reactor vessel to the upper portion of the scrubber at a rate of 100 ml/min. to collect the produced propylene oxide and discharge it therefrom to the outside, while the microorganism-containing reaction mixture was guided to the regenerating vessel at a rate of 20 ml/min., where the microorganisms having decreased methane oxidizability were regenerated, as already mentioned, and were thereafter again returned to the reactor vessel for the production of propylene oxide.

After the initiation of the reaction, a fresh regenerating solution in which the concentration of copper sulfate specified in Table 1 was changed to 1500 µg per liter was supplied at a rate of 120 ml/hr. to make up for decreases in the amount of the solution due to the evaporation of moisture and promote the regeneration of microorganisms, and the reaction mixture containing surplus microorganisms was discharged from the system to the outside. It is noted that the regeneration vessel was controlled to pH 7 with 0.5 M nitric acid. The gas collected with the scrubbers was analyzed by gas chromatography to determine the quantity of the propylene oxide produced. The results are set forth in Table 27.

Table 27

| | Time (hour) Elapsed After Reaction Initiation | | | | | |
|---|---|---|---|---|---|---|
| | 0 | 1 | 24 | 168 | 240 | 288 |
| Microbial Concentration in Reactor (mg/ml) | 2.2 | 2.2 | 2.4 | 2.1 | 2.3 | 2.3 |
| Microbial Activity in Reactor (nmol/min.·mg of microorganism) | 338 | 284 | 178 | 182 | 191 | 176 |
| Microbial Activity in Regenerating Vessel (nmol/min.·mg of microorganism) | 338 | 311 | 220 | 236 | 242 | 230 |
| Amount of Propylene Oxide Produced (mmol/hr.) | - | 3.15 | 2.86 | 2.77 | 2.79 | 2.78 |
| Propylene Oxide Concentration in Regenerating Vessel (mM) | 0 | 0.08 | 0.06 | 0.06 | 0.07 | 0.06 |

As clearly understood from the table, propylene oxide could continuously be produced in a stable state by regenerating the microorganisms in the regenerating vessel. It is noted that the activity of microorganisms was measured in the following manner. The microorganisms were suspended in a 5 mM PIPES buffer at a regulated concentration of 0.5 mg/ml, and 1 ml of the suspension was put in a Mayer's flask of 7 ml in volume. The flask was closed with a rubber stopper, followed by the addition of 2 ml of propylene, and was then vigorously shaken at 45°C for 30 seconds. Subsequently, methanol was added to the flask at a

regulated concentration of 1 mM, which was in turn vigorously shaken at 45 °C for 3 minutes. Afterwards, gas chromatography was applied to determine the quantity of the propylene oxide produced per minute per 1 mg of microorganism.

Comparative Example 19

Example 78 was repeated, except that no methanol was supplied to the regenerating vessel, and only air was fed at a rate of 1.35 liters per minute in place of the mixed methane/air gas. Where the regenerating vessel was not permitted to work, the production of propylene oxide substantially stopped after the lapse of 24 hours. The results are set forth in Table 28.

Table 28

| | Time (hour) Elapsed After Reaction Initiation | | | |
|---|---|---|---|---|
| | 0 | 1 | 7 | 24 |
| Microbial Concentration in Reactor (mg/ml) | 2.3 | 2.3 | 2.1 | 1.5 |
| Microbial Activity in Reactor (nmol/min.·mg of microorganism) | 352 | 161 | 36 | 9 |
| Microbial Activity in Regenerating Vessel (nmol/min.·mg of microorganism) | 358 | 183 | 42 | 9 |
| Amount of Propylene Oxide Produced (mmol/hr.) | - | 2.98 | 0.21 | <0.1 |
| Propylene Oxide Concentration in Regenerating Vessel (mM) | 0 | 0.06 | <0.01 | <0.01 |

Example 79

This example was carried out using the apparatus system shown in Figure 2.

2.4 liters of a medium obtained by mixing together the medium specified in Table 1 except that the concentration of $CuSO_4 \cdot 5H_2O$ was changed to 1000 $\mu$g and the medium specified in Table 2 in a mixing

ratio of 100 : 1.5 were charged into a regenerating vessel B through a sterile filter, and were inoculated with a seed culture of Methylococcus capsulatus NCIB 11132 cultured in a manner similar to that described in Example 78. Air and methane were supplied under 900 r.p.m. agitation to the regenerating vessel at the respective rates of 1200 ml/min. and 230 ml/min. which was maintained at 45°C.

Next, a pump 1 was started to supply the microbial suspension to the upper end of a reactor vessel A at a rate of 10 ml/min. for circulation therethrough. After 24 hours, a pump 2 was actuated to start to supply a 10 v/v % methanol solution to the lower portion of the regenerating vessel at a rate of 200 µl/min. and, at the same time, the amount of methane supplied to the regenerating vessel was decreased from 230 µl/min. to 30 µl/min. After the lapse of further 24 hours, the microbial concentration reached 1.8 mg/min., whereupon the reaction begun to occur.

Ethane was supplied from the reactor vessel through its lower portion at a rate of 320 ml/min. and, at the same time, a pump 3 was actuated to supply an 1 v/v % methanol solution from the upper portion of the reactor vessel at a rate of 40 µl/min. During the reaction, a fresh regenerating solution in which the concentration of copper sulfate specified in Table 1 was changed to 1500 µg/l was supplied at a rate of 120 ml/min. so as to keep constant the amount of the solution contained in the regenerating vessel, and an extra microbial suspension was discharged from within the system to the outside. During the reaction, pH was also adjusted to 7.2 with 0.5 M nitric acid and potassium hydroxide. Moreover, acetaldehyde in the effluent gas collected from the upper portion of the reaction vessel was analyzed by gas chromatography. The results are set forth in Table 29.

Table 29

| | Time (hour) Elapsed After Reaction Initiation | | | | |
|---|---|---|---|---|---|
| | 0 | 1 | 24 | 48 | 72 |
| Microbial Concentration in Reactor (mg/ml) | 1.8 | 1.8 | 1.9 | 2.1 | 2.2 |
| Microbial Activity in Regenerating Vessel (nmol/min.·mg of microorganism) | 298 | 264 | 248 | 232 | 244 |
| Acetaldehyde Concentration in Regenerating Vessel (nmol/min.·mg of microorganism) | 0 | 0.26 | 0.64 | 0.72 | 0.74 |
| Amount of Acetaldehyde Produced (mol/hour) | - | 76 | 85 | 89 | 97 |

Comparative Example 20

Example 79 was repeated, except that the scrubber was removed from the apparatus system of Fig. 2 with the reactor vessel being connected directly to the regenerating vessel, and neither methanol nor methane was supplied to the regenerating vessel. As a result, the amount of the acetaldehyde produced after 24 hours was at most 5 µmol/hour.

Example 80

With the same apparatus system as used in Example 78, Example 78 was repeated, except that 1-butene was used in place of propylene. The results are set forth in Table 30.

Table 30

| | Time (hour) Elapsed After Reaction Initiation | | | | | |
|---|---|---|---|---|---|---|
| | 0 | 1 | 24 | 168 | 240 | 288 |
| Microbial Concentration in Reactor (mg/ml) | 2.1 | 2.0 | 2.3 | 2.4 | 2.3 | 2.3 |
| Microbial Activity in Reactor (nmol/min. mg of microorganism) | 350 | 289 | 198 | 207 | 203 | 207 |
| Microbial Activity in Regenerating Vessel (nmol/min.·mg of microorganism) | 352 | 304 | 254 | 262 | 260 | 268 |
| Amount of 1,2-Butylene Oxide Produced (nmol/min.·mg of microorganism) | - | 1.98 | 1.62 | 1.72 | 1.58 | 1.68 |
| Butylene Oxide Concentration in Regenerating Vessel (mM) | 0 | 0.21 | 0.27 | 0.24 | 0.26 | 0.25 |

Example 81

This example was carried out with the apparatus system shown in Figure 1.

1.8 liters of a medium obtained by mixing together the medium specified in Table 1 except that the concentration of $CuSO_4 \cdot 5H_2O$ was changed to 1000 µg and the medium specified in Table 2 in a mixing ratio of 100 : 1.5 were charged into a regenerating vessel B of 24 liters in volume through a sterile filter.

On the other hand, 50 ml portions of a medium having the same composition, which has been treated through a sterile filter for the removal of infectious microbes, were charged into six Mayer's flasks of 500 ml in volume. One platinum loop of slant-cultured Methylococcus capsulatus NCIB 11132 was inoculated into each flask, which was then closed with a rubber plug, followed by pouring of 100 ml of methane. Culture was subsequently carried out at 45°C for 3 days. The obtained culture solution amounting to 300 ml in all was inoculated into the medium contained in the vessel B. While supplying a mixed methane/air gas of 1 : 4 at a rate of 1.35 liters per minute and carbon dioxide at a rate of 0.05 liter per minute to the vessel B, cultivation was carried out at 45°C for 48 hours with shaking at 1,000 r.p.m. In consequence, the microbial concentration reached 0.7 mg/l.

Next, the microbial suspension was supplied to a reactor vessel A at a rate of 3 liters per hour by means of a pump 1. The amount of the solution contained in the reactor A was controlled to 320 ml by means of a pump 3. The pump 3 was actuated to supply all the amount of the microbial suspension from the reactor A to the upper portion of a scrubber D and return it to the vessel B through the lower portion thereof. Next, the medium specified in Table 31 was supplied to the vessel B at a rate of 1.7 liters/hour, while air and methane were fed to the vessel B at the respective rate of 1.5 liters/min. and 0.27 liters/min. for agitation at 1,500 r.p.m. This condition was maintained for 24 hours.

Table 31

| | |
|---|---|
| Magnesium sulfate$\cdot 7H_2O$ | 1.0 g |
| Potassium nitrate | 1.5 g |
| Calcium chloride | 20 mg |
| $NaMoO_4$ | 1 mg |
| $FeSO_4 \cdot 7H_2O$ | 500 µg |
| $ZnSO_4 \cdot 7H_2O$ | 400 µg |
| $H_3BO_4$ | 15 µg |
| $CoCl_2 \cdot 6H_2O$ | 50 µg |
| $MnCl_2 \cdot 4H_2O$ | 20 µg |
| $NiCl_2 \cdot 6H_2O$ | 10 µg |
| $CuSO_4 \cdot 5H_2O$ | 2000 µg |
| EDTA | 250 µg |

Table 31 (continued)

| | |
|---|---|
| $Na_2PHO_4 \cdot 12H_2O$ | 645 mg |
| $KH_2PO_4$ | 234 mg |
| Fe-EDTA | 7.2 mg |
| Distilled water | 1 liter |

After the microbial concentration had reached 2 mg/ml, the pump 2 was actuated to supply the suspension contained in the reactor A to the upper portion of a scrubber C at a rate of 120 ml per minute. At the same time, all the amount of the effluent gas of methane/air/carbon dioxide gas was guided to the lower portion of the scrubber D, and air was additionally fed at a rate of 2 ℓ/min. from the lower portion of the scrubber D and all the amount of the effluent gas discharged through the upper portion thereof was guided to the lower portion of the scrubber C and discharged through the upper portion thereof. This condition was maintained for further 24 hours. The scrubbers C and D, reactor A and vessel B were all maintained at 45 ° C.

Subsequently, while propylene and air were supplied under agitation at 800 r.p.m. to the reactor A from the lower portion thereof at the respective rates of 230 ml/min. and 340 ml/min., 1 M methanol solution was fed thereto at a rate of 8.5 mmol/hr. to initiate the reaction.

During that reaction, the reaction vessel and the regenerating vessel were kept at pH 7.4 and 8.1, respectively, with 1 M nitric acid. Extra microorganisms were discharged from the system to the outside to keep 18 liters of the amount of the solution contained in the regenerating vessel.

The effluent gas leaving the upper portion of the scrubber C was analyzed by mass spectrometer to determine the quantity of the propylene oxide produced. The results are set forth in Table 32.

Table 32

| | Time (hour) Elapsed After Reaction Initiation | | | | |
|---|---|---|---|---|---|
| | 0 | 1 | 7 | 17 | 27 |
| Microbial Concentration in Reactor (mg/ml) | 2.1 | 2.1 | 2.1 | 1.9 | 1.9 |
| Microbial Activity in Reactor (nmol/min.·mg of microorganism) | 452 | 426 | 416 | 442 | 414 |
| Microbial Activity in Regenerating Vessel (nmol/min.·mg microorganism) | 450 | 261 | 272 | 278 | 304 |
| Amounts of Propylene Oxide Produced (nmol/hour ) | 0 | 8.7 | 8.9 | 8.8 | 8.8 |
| Amount of Propylene Oxide in Regenerating Vessel (mM) | 0 | 0.05 | 0.06 | 0.05 | 0.04 |

As set forth above, propylene oxide could stably be produced even after about 1 month.

Comparative Example 21

The apparatus system was operated for 24 hours in a manner similar to that described in Example 8. However, the supply of methane to the regenerating vessel B was interrupted on the 29th day, and the supply of the regenerating solution (Table 31) to the regenerating vessel B was stopped. The results are set

forth in Table 33.

Table 33

| | |
|---|---|
| Microbial Concentration in Reactor (mg/ml) | 1.7 |
| Microbial Activity in Reactor (nmol/min.•mg of microorganism) | 38 |
| Microbial Activity in Regenerating Vessel (nmol/min.•mg of microorganism) | 45 |
| Amount of Propylene Oxide Produced (nmol/hour) | <0.1 |
| Amount of Propylene Oxide in Regenerating Vessel (mM) | <0.01 |

## Claims

**1.** A method for reactivating an activity of a methane monooxygenase of methane-utilizing bacteria which have partially or totally lost their capability of oxidizing methane comprising the steps of reactivating said bacteria by regenerating them in a reactivation solution containing at least one of the compounds of not less than 10 n mol/minute•mg of cells methane, about 10 to 600 n mol/minute•mg of cells methanol and about 10 to 600 n mol/minute•mg of cells formaldehyde or a mixture thereof, while supplying thereto not less than 1 n mol/minute•mg of cells nitrogen source, not less than 0.02 n mol/minute•mg of cells sulfur source and oxygen.

**2.** The method according to claim 1, wherein a methionine derivative as the nitrogen source and/or the sulfur source is supplied singly or in combination with these sources to the reactivation solution.

**3.** The method according to claim 1 or 2, wherein said methane-utilizing bacteria belong to any one of the genera Methylococcus, Methylomonas, Methylosinus, Methylocystis, Methylobacterium or Methylobacter.

**4.** A method for the continuous production of oxides comprising the steps of contacting methane-utilizing bacteria having a methane monooxygenase in a reactor vessel with alkanes, alkenes or cyclic compounds in the presence of an electron donor, discharging the resulting oxides from the system to the outside, regenerating said methane-utilizing bacteria having a decreased capability of oxidizing methane in a regenerating vessel containing at least one of the compounds of not less than 10 n mol/minute•mg of cells methane, about 10 to 600 n mol/minute•mg of cells methanol and about 10 to 600 n mol/minute•mg of cells formaldehyde or a mixture thereof, while supplying thereto not less than 1 n mol/minute•mg of cells nitrogen source, not less than 0.02 n mol/minute•mg of cells sulfur source and oxygen and returning said methane-utilizing bacteria into the reactor vessel for the further production of oxides.

**5.** The method according to claim 4, wherein benzoic acid or a metal salt thereof is added to the reactor vessel and/or regenerating vessel.

**6.** The method according to claim 5, wherein said benzoic acid or the metal salt thereof is added in an amount of 0.1 to 8 mmol/liter.

**7.** The method according to any one of claims 4 to 6, wherein said methane-utilizing bacteria belong to any one of the genera Methylococcus, Methylomonas, Methylosinus, Methylocystis, Methylobacterium or Methylobacter.

## Patentansprüche

**1.** Verfahren zur Wiederherstellung einer Aktivität einer Methan-Monooxygenase von Methan-verwertenden Bakterien, die ihre Fähigkeit zur Oxidation von Methan teilweise oder vollständig verloren haben, umfassend die Schritte der Reaktivierung der Bakterien durch deren Regeneration in einem Reaktivierungsmedium enthaltend mindestens eine der Verbindungen in einer Menge nicht weniger als 10 nMol/min.•mg pro Zellen Methan, etwa 10 bis 600 nMol/min.•mg pro Zellen Methanol und etwa 10 bis 600 nMol/min.•mg pro Zellen Formaldehyd oder ein Gemisch davon, während nicht weniger als 1

nMol/min.•mg pro Zellen einer Stickstoffquelle, nicht weniger als 0.02 nMol/min.•mg pro Zellen einer Schwefelquelle und Sauerstoff zugefügt werden.

**2.** Verfahren nach Anspruch 1, wobei ein Methionin-Derivat als die Stickstoffquelle und/oder die Schwefelquelle allein oder in Kombination mit diesen Quellen zur Reaktivierungslösung zugefügt werden.

**3.** Verfahren nach Anspruch 1 oder 2, wobei die Methan-verwertenden Bakterien zu einer der Gattungen Methylococcus, Methylomonas, Methylosinus, Methylocystis, Methylobacterium oder Methylobacter gehören.

**4.** Verfahren zur kontinuierlichen Herstellung von Oxiden, umfassend die Schritte des in Berührungbringens von Methan-verwertenden Bakterien, die eine Methan-Monooxygenase aufweisen, in einem Reaktionsgefäß mit Alkanen, Alkenen oder cyclischen Verbindungen in Gegenwart eines Elektronendonors, die Entnahme der erhaltenen Oxide aus dem System nach außen, die Regenerierung der Methan-verwertenden Bakterien, die eine verringerte Fähigkeit zur Oxidierung von Methan aufweisen, in einem Regenerationsgefäß enthaltend mindestens eine der Verbindungen in einer Menge von nicht weniger als 10 nMol/min.•mg pro Zellen Methan, etwa 10 bis 600 nMol/min.•mg pro Zellen Methanol und etwa 10 bis 600 nMol/min.•mg pro Zellen Formaldehyd oder ein Gemisch davon, während nicht weniger als 1 nMol/min.•mg pro Zellen einer Stickstoffquelle, nicht weniger als 0.02 nMol/min.•mg pro Zellen einer Schwefelquelle und Sauerstoff zugefügt werden, und Rückführung der Methan-verwertenden Bakterien in das Reaktionsgefäß zur weiteren Herstellung von Oxiden.

**5.** Verfahren nach Anspruch 4, wobei Benzoesäure oder ein Metallsalz davon zum Reaktionsgefäß und/oder Regenerationsgefäß zugesetzt werden.

**6.** Verfahren nach Anspruch 5, wobei die Benzoesäure oder das Metallsalz davon in einer Menge von 0.1 bis 8 mMol/l zugesetzt werden.

**7.** Verfahren nach einem der Ansprüche 4 bis 6, wobei die Methan-verwertenden Bakterien zu einer der Gattungen Methylococcus, Methylomonas, Methylosinus, Methylocystis, Methylobacterium oder Methylobacter gehören.

**Revendications**

**1.** Procédé de réactivation d'une activité méthane mono-oxygénase de bactéries utilisant le méthane qui ont partiellement ou totalement perdu leur aptitude à oxyder le méthane, lequel comprend les étapes consisant à réactiver lesdites bactéries en les régénérant dans une solution de réactivation contenant au moins l'un des composés suivants: au moins 10 nmoles de méthane/minute•mg de cellules, environ 10 à 600 nmoles de méthanol/minute•mg de cellules et environ 10 à 600 nmoles de formaldéhyde/minute•mg de cellules, ou un mélange de ces composés, en ajoutant au moins 1 nmole/minute•mg de cellules d'une source d'azote, au moins 0,02 nmole/minute•mg de cellules d'une source de soufre et de l'oxygène.

**2.** Procédé selon la revendication 1 dans lequel un dérivé de la méthionine est fourni en tant que source d'azote et/ou source de soufre seul ou en combinaison avec ces sources dans la solution de réactivation.

**3.** Procédé selon la revendication 1 ou 2 dans lequel lesdites bactéries utilisatrices de méthane appartiennent à l'un des genres suivants: Méthylococcus, Méthylomonas, Méthylosinus, Méthylocystis, Méthylobactérium ou Méthylobacter.

**4.** Procédé pour la production continue d'oxydes comprenant les étapes consistant à mettre en contact des bactéries utilisatrices de méthane possédant une méthane mono-oxygènase dans un récipient de réaction avec des alcanes, des alcènes ou des composés cycliques en présence d'un donneur d'électrons, à déverser les oxydes résultants à l'extérieur du système, à régénérer lesdites bactéries utilisatrices de méthane dont l'aptitude à oxyder le méthane est réduite dans un récipient de régénération contenant au moins l'un des composés suivants: au moins 10 nmoles de méthane/minute•mg de cellules, environ 10 à 600 nmoles de méthanol/minute•mg de cellules et

environ 10 à 600 nmoles de formaldéhyde/minute•mg de cellules, ou un mélange de ces composés, en ajoutant au moins 1 nmole/minute•mg de cellules d'une source d'azote, au moins 0,02 nmole/minute•mg de cellules d'une source de soufre et de l'oxygène et à replacer lesdites bactéries utilisatrices de méthane dans le récipient de réaction en vue d'une nouvelle production d'oxydes.

**5.** Procédé selon la revendication 4 dans lequel l'acide benzoïque ou un sel métallique de celui-ci est ajouté dans le récipient de réaction et/ou dans le récipient de régénération.

**6.** Procédé selon la revendication 5 dans lequel l'acide benzoïque ou son sel métallique est ajouté en une quantité de 0,1 à 8 mmoles/litre.

**7.** Procédé selon l'une quelconque des revendications 4 à 6 dans lequel lesdites bactéries utilisatrices de méthane appartiennent à l'un des genres suivants: Méthylococcus, Méthylomonas, Méthylosinus, Méthylocystis, Méthylobactérium ou Méthylobacter.

FIG. 1

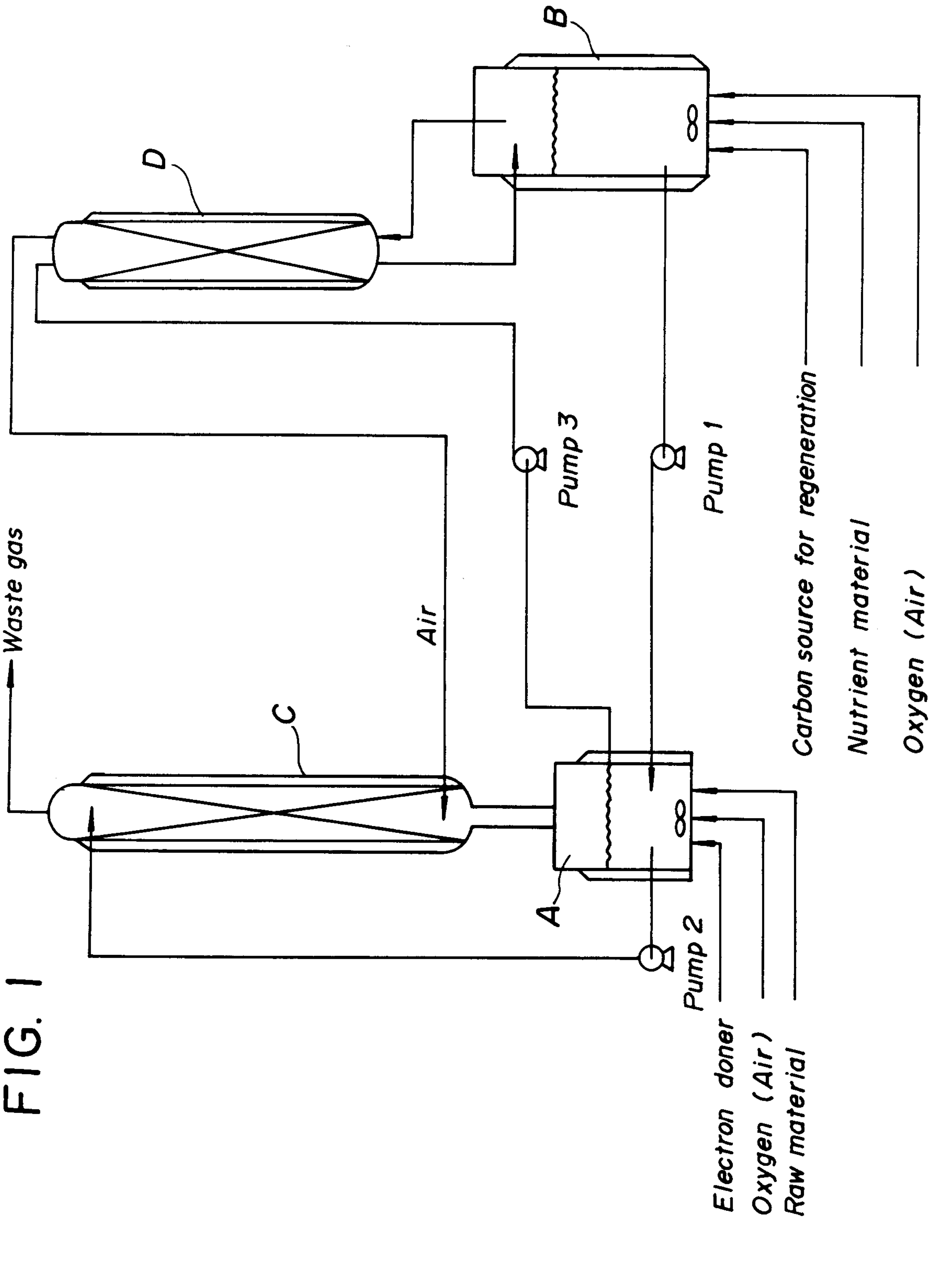

B
D
C
A
Waste gas
Air
Pump 1
Pump 2
Pump 3
Carbon source for regeneration
Nutrient material
Oxygen (Air)
Electron doner
Oxygen (Air)
Raw material

FIG. 2

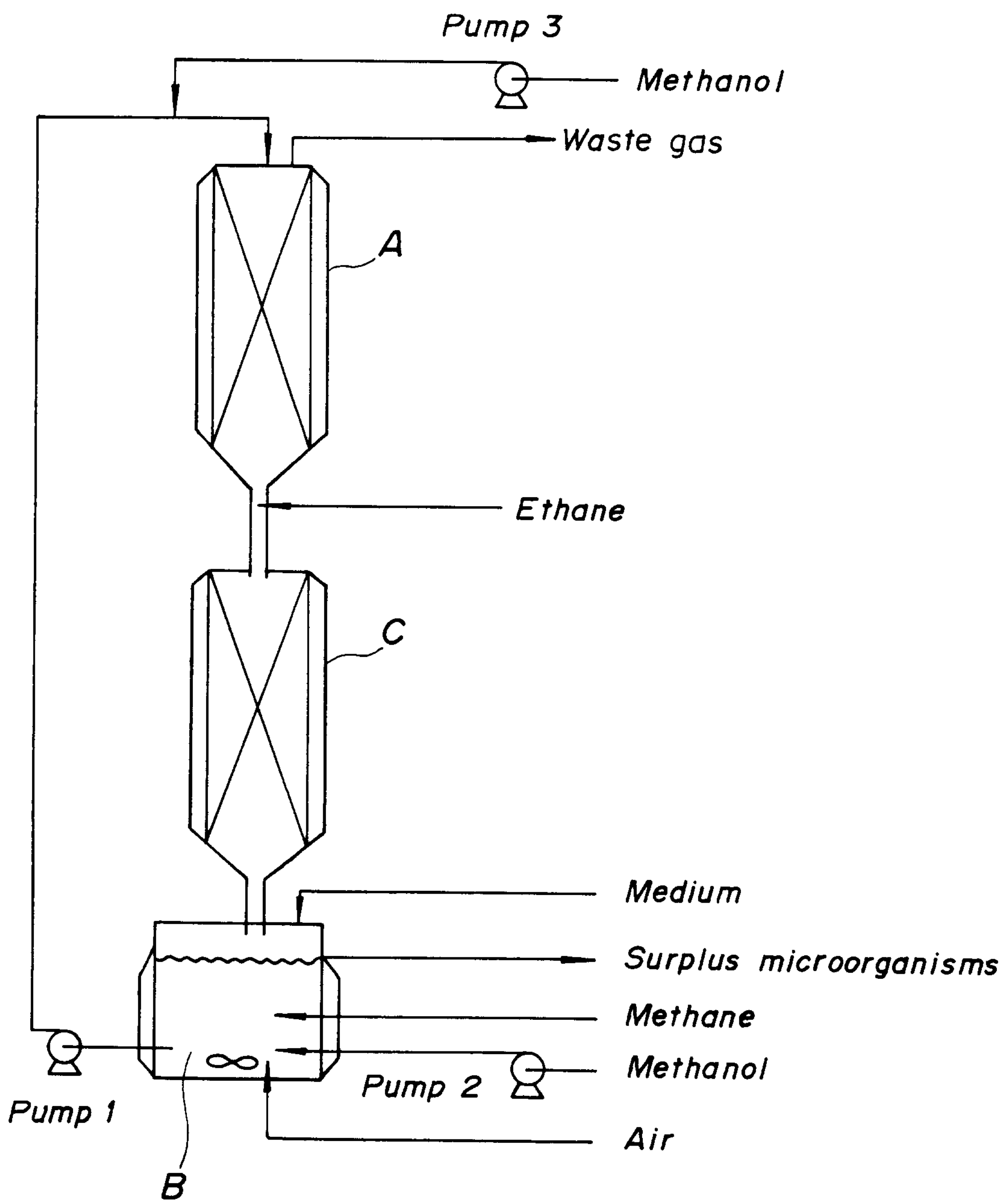
Pump 3
Methanol
Waste gas
A
Ethane
C
Medium
Surplus microorganisms
Methane
Methanol
Pump 2
Air
Pump 1
B